# EUROPEAN PATENT SPECIFICATION

(11) **EP 1 706 063 B1**
(45) Date of publication and mention of the grant of the patent: **30.10.2013**
(21) Application number: 05711539.6
(22) Date of filing: 18.01.2005
(51) Int. Cl.: A61F 2/01

(54) **SHEATH FOR USE WITH AN EMBOLIC PROTECTION FILTERING SYSTEM**
HÜLLE FÜR EINEN FILTER ZUM SCHUTZ VOR EMBOLI
GAINE UTILISEE AVEC UN SYSTEME DE FILTRAGE A PROTECTION EMBOLIQUE

(30) Priority: 20.01.2004 US 762646
(43) Date of publication of application: 04.10.2006
(73) Proprietor: Boston Scientific Limited, St. Michael, Barbados, West Indies (BB)
(72) Inventor: LOWE, Brian J., Zimmerman, Minnesota 55398 (US); SALAHIEH, Amr, Saratoga, California 95070 (US); VRBA, Anthony C., Maple Grove, Minnesota 55311 (US)
(74) Representative: Vossius & Partner
(86) International application number: PCT/US2005/001448
(87) International publication number: WO 2005/072646

(56) References cited:
- EP-A- 0 947 168
- EP-A- 1 149 566
- WO-A-2004/030577
- WO-A1-2004/021928
- US-A- 6 001 118
- US-A1- 2002 183 781
- US-A1- 2003 004 537
- US-A1- 2004 044 359
- US-A1- 2004 181 237

## Description

### Field of the Invention

The present invention pertains to embolic protection filtering devices. More particularly, the present invention pertains to sheaths for delivering and retrieving embolic protection filtering devices.

### Background

Heart and vascular disease are major problems in the United States and throughout the world. Conditions such as atherosclerosis result in blood vessels becoming blocked or narrowed. This blockage can result in lack of oxygenation of the heart, which has significant consequences since the heart muscle must be well oxygenated in order to maintain its blood pumping action.

Occluded, stenotic, or narrowed blood vessels may be treated with a number of relatively non-invasive medical procedures including percutaneous transluminal angioplasty (PTA), percutaneous transluminal coronary angioplasty (PTCA), and atherectomy. Angioplasty techniques typically involve the use of a balloon catheter. The balloon catheter is advanced over a guidewire such that the balloon is positioned adjacent a stenotic lesion. The balloon is then inflated and the restriction of the vessel is opened. During an atherectomy procedure, the stenotic lesion may be mechanically cut away from the blood vessel wall using an atherectomy catheter.

During angioplasty and atherectomy procedures, embolic debris can be separated from the wall of the blood vessel. If this debris enters the circulatory system, it could block other vascular regions including the neural and pulmonary vasculature. During angioplasty procedures, stenotic debris may also break loose due to manipulation of the blood vessel. Because of this debris, a number of devices, termed embolic protection devices, have been developed to filter out this debris.

Document US 2003/0004537 discloses a retrieval catheter for embolic protection devices and represents the closest priort art. Document EP 1536740 is state of the art according Article 54(3) EPC.

### Brief Summary

The present invention pertains to sheaths that may be used for delivering and/or retrieving embolic protection filtering devices. In at least some embodiments, the sheath may include a proximal portion and a distal portion. The distal portion may be expandable and may include a bulbous member. Additionally, the sheath may include structural support in a number of different forms. These and other structural features and characteristics are described in more detail below.

### Brief Description of the Drawings

Figure 1 is a partial cross-sectional plan view of an example sheath;
Figure 1A is a partial cross-sectional plan view of the sheath shown in Figure 1 where the mouth of the sheath is enlarged;
Figure 1B is a partial cross-sectional plan view of the sheath shown in Figure 1 where a filter is disposed in the sheath;
Figure 2 is a partially cut-away side view of another example sheath;
Figure 3 is a detail view of an example braided portion;
Figure 3A is a cross-sectional view of an alternative example of a fiber;
Figure 4 is a cross-sectional view of another example sheath;
Figure 5 is a cross-sectional view of another example sheath;
Figure 6 is a cross-sectional view taken through line 6—6;
Figure 7 is an alternative cross-sectional view of another example bulbous member;
Figure 8 is an alternative cross-sectional view of another example bulbous member;
Figure 9 is an alternative cross-sectional view of another example bulbous member;
Figure 10 is an alternative cross-sectional view of another example bulbous member;
Figure 11 is an alternative cross-sectional view of another example bulbous member;
Figure 12 is an alternative cross-sectional view of another example bulbous member;
Figure 13 is an alternative cross-sectional view of another example bulbous member;
Figure 14 is an alternative cross-sectional view of another example bulbous member;
Figure 15 is a perspective view of another example sheath; and
Figure 16 is a perspective view of another example sheath.

### Detailed Description

The following description should be read with reference to the drawings wherein like reference numerals indicate like elements throughout the several views. The detailed description and drawings illustrate example embodiments of the claimed invention.

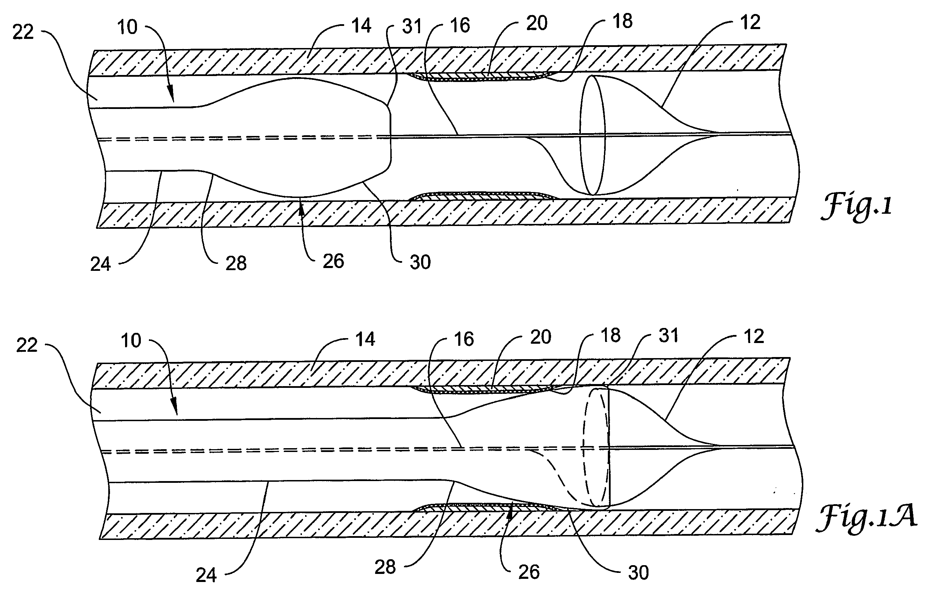

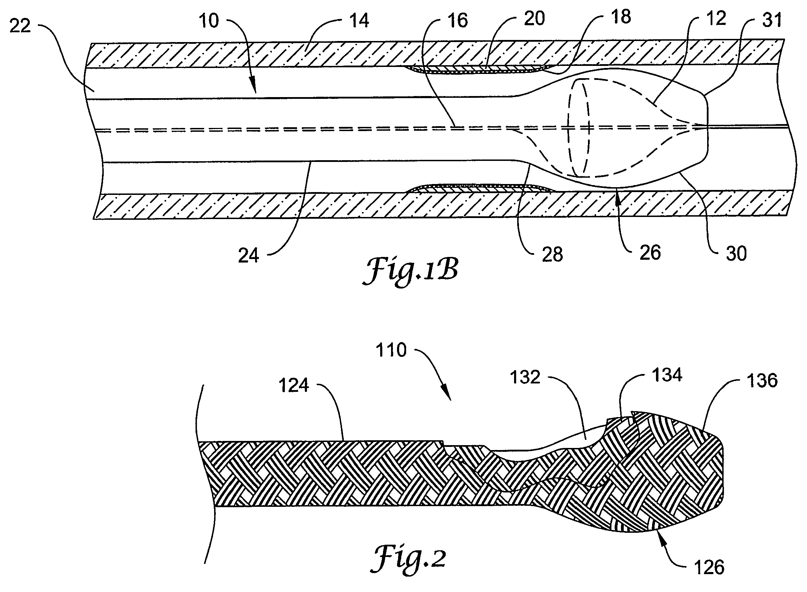

Figure 1 is partial cross-sectional plan view of an example sheath 10 that may be used to facilitate the delivery and/or retrieval of an embolic protection filter 12. When used for retrieval of filter 12, sheath 10 may be advanced through a blood vessel 14 over a guidewire 16 or other medical device having filter 12 coupled thereto. Further distal advancement of sheath 10 may allow filter 12 to become collapsed and disposed within sheath 10. Sheath 10 and filter 12 can then be proximally retracted from vessel 14. When sheath 10 is used for delivery, filter 12 may be collapsed and disposed within sheath 10, sheath 10 can be advanced through the vasculature to an appropriate location, and sheath 10 can be proximally retracted or otherwise configured so that filter 12 expands within vessel 14. The remaining discussion focuses more heavily on some of the characteristics, features, and uses of sheath 10 pertaining to filter retrieval. However, it can be appreciated that a number of these features may analogously apply to filter delivery.

Frequently, intravascular procedures may involve the placement of a stent 18 in blood vessel 14 to open or expand a stenosis 20. It may be desirable for the clinician to include the use of filter 12 when performing the intravascular stent placement procedure. Accordingly, it may be necessary for the clinician to retrieve filter 12 after stent 18 has be placed. Because it may be desirable for a retrieval device to have the greatest possible outside diameter in order to allow the filter to be disposed therein, and because this large outside diameter may cause the retrieval device to contact stent 18, it is possible that the retrieval device may disrupt the position of stent 18. Disrupting the position of stent 18 may undermine its function and efficacy. Therefore, it may be desirable to use retrieval devices that are configured so as to be less likely to disrupt stent 18.

Retrieval may often include disposing a portion of the filter in the retrieval device. However, because the filter may be enlarged due to the amount of debris it is holding, the filter itself may catch on stent 18. Therefore, it may be desirable for a retrieval device to be adapted and configured to fully encapsulate the filter, thereby eliminating the possibility that the filter may disrupt stent 18.

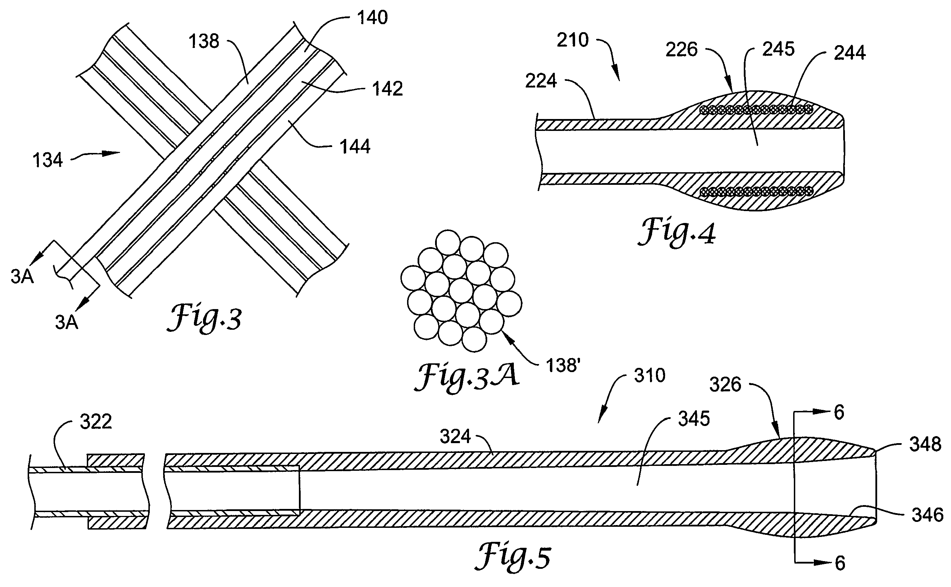

Sheath 10 is an example of one such device. In at least some embodiments, sheath 10 may include a proximal region 22, a distal region 24, and one or more lumens (not shown in Figure 1, but some examples of lumens that may be appropriate are illustrated in Figures 4 and 5). Distal region 24 may include a bulbous region or member 26. Bulbous region 26 may be shaped or otherwise configured to have one or more gradual steps in outside diameter. The transition in outside diameter may be from the generally smaller outside diameter adjacent distal region 24 to the generally larger outside diameter adjacent bulbous region 26. For example, bulbous region 26 may include a proximal tapered surface 28 and a distal tapered surface 30. Tapered surfaces 28/30 may allow sheath 10 to more easily pass stent 18 in either the proximal or distal direction (depending on whether sheath 10 is being proximally retracted or distally advanced along guidewire 16) by allowing the transition in outside diameter to be more gradual than other devices having a blunted end, which could "catch" on stent 18 and disrupt it.

Distal region 24 may be adapted for having filter 12 disposed therein, for example in a lumen extending through distal region 24. In at least some embodiments, distal region 24 is appropriately sized and configure for having filter 12 fully encapsulated therein during retrieval of filter 12. Additionally, because a substantial amount of debris may accumulate and become contained within filter 12, distal region 24 of sheath 10 may be subjected to a relatively large force (e.g., due to the size or weight of filter 12) when attempting to encapsulate or otherwise dispose filter 12 within sheath 10. This force, if not properly absorbed, could lead to the distal end of a retrieval device being expanded, collapsed, or even prolapsed, which may increase the likelihood that the retrieval device may contact and disrupt stent 18. Therefore, it may be desirable for distal region 24 of sheath 10 to be structurally reinforced, so as to more easily absorb these forces produced when encapsulating or otherwise disposing filter 12 within sheath 10. In at least some embodiments, bulbous region 26 may have an increased thickness, which may add additional structural support to distal region 24 of sheath 10.

In at least some embodiments, sheath 10 may include an expandable or enlargeable distal mouth 31 disposed adjacent distal region 24. The expandability of mouth 31 may be at least partially due to the material composition of sheath 10. For example, the materials used to construct sheath 10 (e.g., adjacent mouth 31) may be substantially elastic and/or include elastomeric polymers. Some examples of suitable elastic materials may include elastomeric polyamides, polyether-ester elastomers, and the like as well as other materials including those listed herein. Mouth 31 may be configured to reversibly enlarge in order for sheath 10 to engulf or otherwise accommodate filter 12 therein. For example, Figure 1A illustrates sheath 10 where it has been distally advanced toward filter 12 and where mouth 31 is enlarged to an expanded configuration or position so as to allow filter 12 to pass therein. Further distal advancement of sheath 10 allows filter 12 to become essentially engulfed within distal region 24 of sheath 10 as shown in Figure 1B. After sheath 10 has passed over filter 12, mouth 31 can close or otherwise resume its previous non-expanded or basal configuration.

Figure 2 is a partially cut away perspective view of another example sheath 110, that is similar to other sheaths described herein, where distal region 124 (and bulbous member 126) may be reinforced by including one or more braids. For example, sheath 110 may include an inner member 132, which may or may not include any braiding. A first braided layer 134 may be disposed on inner member 132. A second braided layer 136 (as well as additional braided or other layers) may be disposed on first braided layer 134. The exact arrangement and position of layers included in sheath 110 may vary. For example, the various braided layers may be disposed along essentially the entire length of sheath 110 (including proximal region 122) or any region or combination of regions including at bulbous member 126 and, in some embodiments, at bulbous member 126 only. Additionally, the layers or may be disposed intermittently so that the various regions may include differing numbers or configurations of layers. It can be appreciated that any of the other structural features or other characteristics of any of the other sheaths described herein, for example having an expandable distal mouth similar to what is disclosed above, may be included with sheath 110.

Figure 3 is a more detailed view of an example braided layer (in this case braided layer 134) that is appropriate for any of the embodiments of sheaths disclosed herein. Braided layer 134 may include a plurality of fibers, for example, a first fiber 138, a second fiber 140, a third fiber 142, and a fourth fiber 144. Of course, including four fibers in braided layer 134 is not intended to be limiting as any appropriate number of fibers may be utilized.

Fibers 138/140/142/144 can be made of any suitable materials including metals, metal alloys, polymers, or the like, or combinations or mixtures thereof. Some examples of suitable metals and metal alloys include stainless steel, such as 304v stainless steel; nickel-titanium alloy, such as nitinol, nickel-chromium alloy, nickel-chromium-iron alloy, cobalt alloy, or the like; or other suitable material. Some examples of suitable polymers may include polytetrafluoroethylene (PTFE), ethylene tetrafluoroethylene (ETFE), fluorinated ethylene propylene (FEP), polyurethane, polypropylene (PP), polyvinylchloride (PVC), polyether-ester (for example a polyether-ester elastomer such as ARNITEL® available from DSM Engineering Plastics), polyester (for example a polyester elastomer such as HYTREL® available from DuPont), polyamide (for example, DURETHAN® available from Bayer or CRISTAMID® available from Elf Atochem), elastomeric polyamides, block polyamide/ethers, polyether block amide (PEBA, for example available under the trade name PEBAX®), silicones, polyethylene, Marlex high-density polyethylene, Marlex low-density polyethylene, linear low density polyethylene (for example REXELL®), polyethylene terephthalate (PET), polyetheretherketone (PEEK), polyimide (PI), polyetherimide (PEI), polyphenylene sulfide (PPS), polyphenylene oxide (PPO), polysulfone, nylon, perfluoro(propyl vinyl ether) (PFA), other suitable materials, or mixtures, combinations, or copolymers thereof. In some embodiments, fibers 138/140/142/144 can include a liquid crystal polymer (LCP) blended with other polymers to enhance torqueability.

Fibers 138/140/142/144, or portions thereof, may also be doped with or otherwise include a radiopaque material. Radiopaque materials are understood to be materials capable of producing a relatively bright image on a fluoroscopy screen or another imaging technique during a medical procedure. This relatively bright image aids the user of sheath 10 in determining its location. Some examples of radiopaque materials can include, but are not limited to, gold, platinum, palladium, tantalum, tungsten alloy, plastic material loaded with a radiopaque filler, and the like.

In some embodiments, braided layer 134 may include combinations of fibers that include differing materials. For example, first fiber 138 may be a platinum wire and the remaining fibers 140/142/144 may have a polymeric composition. This configuration of fibers may be desirable for a number of reasons. For example, including a platinum first fiber 138 allows sheath 110 to be visualized using conventional fluoroscopy techniques. Additionally, the choice of polymer used for remaining fibers 140/142/144 can be selected according to the characteristics desired for sheath 110. For example, relatively flexible polymers may be used in embodiments where flexibility is desired.

The shape, thickness, and other characteristics of fibers 138/140/142/144 may also vary. For example, any single or combination of fibers 138/140/142/144 may include a generally round wire, a generally flat ribbon, other shapes, and the like. Similarly, the diameter or thickness of fibers 138/140/142/144 may vary. Some embodiments include sets of fibers having the same diameter while other embodiments include sets of fibers having differing diameters. Additionally, some generally thick fibers (e.g., which may include metal and radiopaque fibers such as fiber 138) can be replaced by a set of micro-cables 138' as illustrated in Figure 3A. These micro-cables 138' are sets of wires that, when considered together as a unit, have a cross-sectional diameter that approximates fiber 136. However, micro-cables 138' may have physical characteristics (e.g., flexibility) that more closely resemble those of the individual wires. Thus, this feature may help maintain flexibility by replacing a thick, relatively stiff fiber with a set of micro-cables 138' that, individually, may be relatively flexible.

Manufacturing of sheath 110 may include convention braiding techniques and equipment. In some embodiments, multiple fibers (e.g., fibers 138/140/142/144) may be added to a braiding bobbin, which may create a "multi-ended" braid. For example, fibers 138/140/142/144 can be added to the braiding bobbin and braided onto an appropriate substrate. The appropriate substrate may vary and could include a mandrel, mold, tube similar to inner member 132, and the like. It should be noted that some embodiments, like one of the examples described above where first fiber 138 is a platinum wire, it may still be appropriate to add all of the fibers 138/140/142/144 to a single bobbin, for example, by simply adding a platinum wire (e.g., fiber 138) to a bobbin having multiple polymeric fibers (e.g., fibers 140/142/144) to create a multi-ended braid that includes fibers of differing materials.

Another example sheath 210 is illustrated in Figure 4. Sheath 210 is similar to other sheaths described herein, except that it includes a frame or coil 244. In some embodiments, coil 244 may be disposed adjacent bulbous region 226 and may provide structural support. However, coil 244 may be disposed at any appropriate location including distal region 224, the proximal region (not shown), combinations of regions, or the entire length of sheath 210. Other types of frame structures may be substituted with coil 244. For example, appropriate substitutes for coil 244 may include a branching or non-branching frame, a leaf spring shaped frame, a stent-shaped frame, or other suitable structures. Figure 4 also shows that a lumen 245 may extend through sheath 210 as well as any of the other sheaths described herein. Lumen 245 may allow sheath 210 to advance along guidewire 16 and have at least a region that is sized appropriately for having filter 12 disposed therein.

Another example sheath 310 is illustrated in Figure 5. Sheath 310 is similar to other sheaths described herein and may include proximal region 322, distal region 324, , bulbous region 326, and lumen 345. However, distal region 324 and proximal region 322 may comprise separate structures that are attached by any one of a number of techniques. For example, proximal region 322 and distal region 324 may be attached by adhesive bonding with or without the inclusion of appropriate tie layers. Alternatively, distal region 324 may be molded (e.g., insert molded) onto proximal region 322. Essentially any other appropriate molding or bonding technique may be used.

In some embodiments, proximal region 322 may comprise a typical retrieval sheath. According to this embodiment, lumen 345 may be in fluid communication with the lumen of the typical retrieval device. Distal region 324 may be a structure that is attachable to these typical retrieval devices in order to provide sheath 310 with desirable characteristics such as those described herein. This feature allows distal region 324 to be similar to an "adaptor" or other refining device that can be used to provide existing devices with at least some of the desirable features attributable to distal region 324.

The material composition of distal region 324 (as well as any of the componets of sheath 310) may including any of the materials listed herein including metals, metal-alloys, and polymers. For example, distal region 324 may be comprised of silicone of essentially any appropriate durometer. According to this embodiment, distal region 324 may be adhesively bonded to proximal region 322 using appropriate adhesives or tie layers. Alternatively, distal region 324 may be comprised of injection-moldable materials such as DURAFLEX™, linear block copolymer such as KRATON™, SUPERFLEX™, and the like.

Distal region 324 may also include any of the other structural elements described herein, for example, to provide structural support to distal region 324. For example, distal region 324 may include one or more braided layers, a coil, a frame, a stent-like structure, and the like. Distal region 324 may also include bulbous region 326. In some embodiments, bulbous region 326 may provide distal region 324 with additional structural support by increasing the wall thickness of distal region 324. Additionally, bulbous region 326 or regions adjacent it may have a conical or funnel shaped inside diameter surface 346, which may make it easier for filter 12 to pass into distal region 324. Additionally, distal region 324 and/or bulbous region 326 may include a rounded or generally atraumatic distal end 348.

Additional structural strength may also include varying the wall thickness of distal region 324. For example, it can be seen in Figure 5 that distal region 324 may include lumen 345 defining an inside diameter of distal region 324. In some embodiments, variation in the inside diameter of distal region 324 correspond to variation in the wall thickness of distal region 324. According to this embodiment, as the inside diameter decreases, the wall thickness of distal region 324 increases. Changes in wall thickness, similar to what is described above, may alter the compressive strength of distal region 324.

In some embodiments, the inside diameter of distal region 324 may gradually taper in the proximal direction. This may correspond with increased wall thickness for distal region 324 in the proximal direction. This feature may help to provide an increased compressive strength adjacent some of the proximal positions of distal region 324, including positions where filter 12 may eventually become disposed. Additionally, because the taper may be gradual, weak points that might otherwise have been created by pronounced steps in inside diameter can be reduced.

Bulbous member 326 may be defined or otherwise formed in a number of ways. For example, bulbous member 326 may be defined during the molding of distal region 324. Alternatively, bulbous member 326 may be defined by molding or otherwise coupling it to distal region. For example, bulbous member 326 may be defined by adhesively bonding or insert molding it to distal region 324.

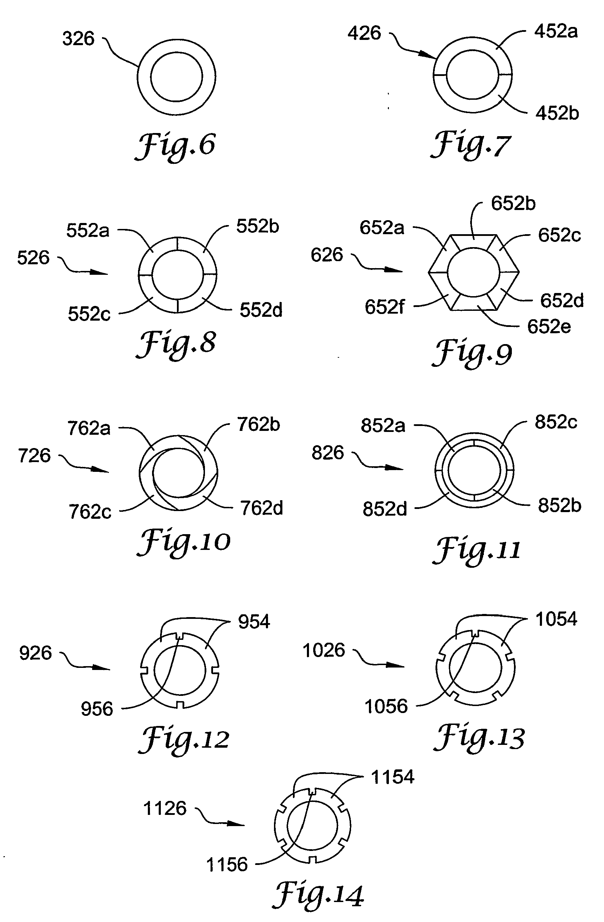

Regardless of how bulbous member 326 is defined, it may vary in its shape and configuration. Figures 6-14 illustrate cross-sectional views of some examples of the various shapes and configurations that may be for bulbous member 326. For example, Figure 6 shows that bulbous member 326 as being a single piece that may be integral with distal region 324 or be molded to or adhesively bonded to distal region 324. On the other hand, bulbous member 326 may include a plurality of subunits. For example, Figure 7 shows an alternative bulbous member 426 having two subunits 452a/b, Figure 8 shows bulbous member 526 having four subunits 552a/b/c/d, and Figure 9 shows bulbous member 626 including six subunits 652a/b/c/d/e/f. It can be appreciated that any number of subunits may be included in the various embodiments of the invention.

Figures 10 and 11 show that the subunits may have varying arrangements. For example, bulbous member 726 may include subunits 752a/b/c/d arranged in a swirling pattern as shown in Figure 10. Alternatively, bulbous member 826 includes subunits 852a/b/c/d arranged on opposite sides and on top of one another as in Figure 11.

Figures 12-14 illustrate other example bulbous members that include ridges and voids. The number and arrangement of the ridges and voids may vary. For example, bulbous member 926 of Figure 12 includes four ridges 954 and four voids 956 spaced between ridges 954. Similarly, Figure 13 illustrates bulbous member 1026 having five ridges 1054 and five voids 956. Finally, Figure 14 illustrates bulbous member 1126 having six ridges 1154 and six voids 1156. Of course the number and arrangement of ridges and voids is not intended to be limited as the number and arrangement may vary from what is shown.

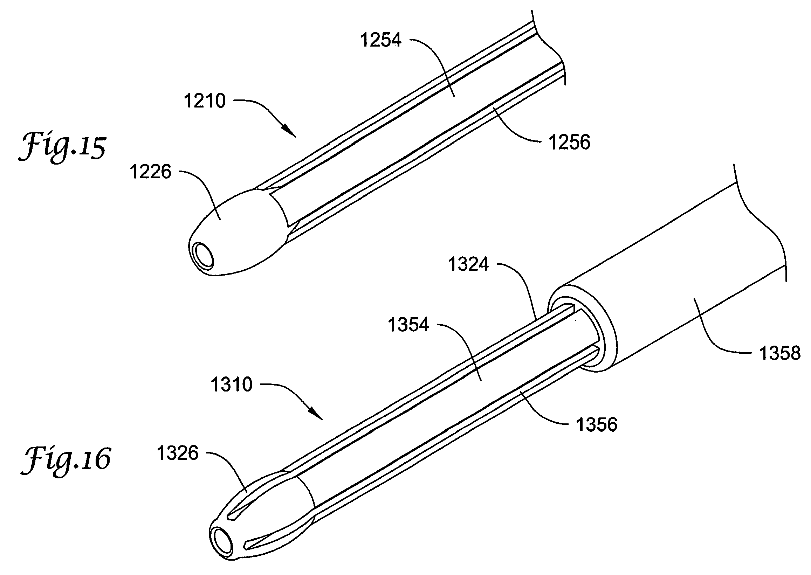

In some embodiments, other portions of the sheath may include ridges and voids. For example, Figure 15 illustrates another example sheath 1210 that includes ridges 1254 and voids 1256 disposed proximally of bulbous member 1226. The combination of ridges 1254 and voids 1256 in sheath 1210 (as well as other structures disclosed herein) may be desirable by allowing sheath to expand adjacent voids 1256 while ridges 1254 impart the desired column strength for sheath 1210. According to this embodiment, including ridges 1254 and voids 1256 may allow sheath 1210 to more easily expand to "swallow" filter 12, while maintaining its column strength and integrity.

Another example sheath 1310 is shown in Figure 16. Sheath 1310 is similar to sheath 1210 (and other described herein), except that ridges 1354 and voids 1356 extend along bulbous member 1326 as well as positions proximally thereof. This feature may be desirable for reasons described above as well as other reasons. For example, including ridges 1354 and voids 1356 proximally of bulbous member 1326 may allow for greater expansion of sheath 1310, which may allow filter 12 to more easily become encapsulated or otherwise disposed therein.

Sheath 1310 may also include a proximal member 1358. In some embodiments, proximal member 1358 may be configured to attach to a retrieval device, similar to what is shown in Figure 5. According to this embodiment, distal region 1324 may have a lower profile than proximal member 1358, which may improve its ability to cross a stent, and include ridges 1354 and voids 1356 so as to be expandable to accommodate filter 12. Proximal member 1358 may then extend proximally from distal region 1324 and be attachable to a retrieval device. Alternatively, proximal member 1358 may include the proximal region of sheath 1310. Accordingly, proximal member 1358 may be configured to extend proximally outside the body of a patient when using sheath 1310 to retrieve filter 12.

It should be understood that this disclosure is, in many respects, only illustrative. Changes may be made in details, particularly in matters of shape, size, and arrangement of steps without exceeding the scope of the invention. The invention's scope is, of course, defined in the language in which the appended claims are expressed.

## Claims

1. A device for use with an embolic protection filter (12), comprising:
an elongate sheath (10) having a proximal region (22), a distal region (24), a lumen extending at least partially therethrough, and a distal mouth (31) disposed adjacent the distal region;
wherein the distal mouth is expandable and is adapted to shift between a basal configuration and an enlarged configuration; **characterized in that**
the distal region includes a bulbous member (26) in the basal configuration.

2. The device of claim 1, wherein the bulbous member includes a tapered proximal edge and a tapered distal edge.

3. The device of claim 1, wherein at least a portion of the distal region has an increased thickness.

4. The device of claim 3, wherein the portion of the distal region that has an increased thickness is disposed adjacent the bulbous member.

5. The device of claim 1, wherein the lumen adjacent the distal region is flared so that the sheath has a first inside diameter adjacent the proximal region and a second inside diameter adjacent the distal region, the second inside diameter being greater than the first inside diameter.

6. The device of claim 1 or 5, wherein the proximal region of the tube is defined by a first tubular shaft and the distal region of the tube is defined by a second tubular shaft attached to the first shaft.

7. The device of claim 1 or 5, wherein the sheath includes a braid.

8. The device of claim 7, wherein the braid is disposed adjacent the distal region.

9. The device of claim 7, wherein the braid is disposed adjacent the bulbous member.

10. The device of claim 7, wherein the braid includes a plurality of fibers that are braided together, and wherein at least one of the fiber includes a radiopaque material.

11. The device of claim 1, wherein the bulbous member is integral with the distal region.

12. The device of claim 1, wherein the bulbous member includes a plurality of subunits.

13. The device of claim 1 or 5, wherein the sheath includes one or more longitudinal grooves.

14. The device of claim 13, wherein the one or more grooves are disposed adjacent the bulbous member.

15. The device of claim 13, wherein the one or more grooves extend proximally of the bulbous member.

16. The device of claim 1, wherein the distal region includes a support coil.

## Patentansprüche

1. Vorrichtung zur Verwendung mit einem Embolieschutzfilter (12), mit:
einer langgestreckten Hülle (10) mit einem proximalen Bereich (22), einem distalen Bereich (24), einem Lumen, das sich mindestens teilweise durch diese erstreckt, und einer distalen Öffnung (31). die sich in Nachbarschaft zum distalen Bereich befindet,
wobei die distale Öffnung expandierbar und geeignet ist, sich zwischen einer Basiskonfiguration und einer vergrößerten Konfiguration zu verändern, **dadurch gekennzeichnet, dass**
der distale Bereich in der Basiskonfiguration ein wulstförmiges Teil (26) aufweist.

2. Vorrichtung nach Anspruch 1, wobei das wulstförmige Teil eine angeschrägte proximale Kante und eine angeschrägte distale Kante aufweist.

3. Vorrichtung nach Anspruch 1, wobei mindestens ein Abschnitt des distalen Bereichs eine erhöhte Dicke hat.

4. Vorrichtung nach Anspruch 3, wobei der Abschnitt des distalen Bereichs, der eine erhöhte Dicke hat, sich in Nachbarschaft zu dem wulstförmigen Teil befindet.

5. Vorrichtung nach Anspruch 1, wobei das Lumen in Nachbarschaft zu dem distalen Bereich aufgeweitet ist, so dass die Hülle einen ersten Innendurchmesser in Nachbarschaft zu dem proximalen Bereich und einen zweiten Innendurchmesser in Nachbarschaft zu dem distalen Bereich hat, wobei der zweite Innendurchmesser größer ist als der erste Innendurchmesser.

6. Vorrichtung nach Anspruch 1 oder 5, wobei der proximale Bereich der Röhre durch einen ersten röhrenförmigen Schaft definiert ist und der distale Bereich des Röhre durch einen zweiten röhrenförmigen Schaft definiert ist, der am ersten Schaft angebracht ist.

7. Vorrichtung nach Anspruch 1 oder 5, wobei die Hülle ein Geflecht aufweist.

8. Vorrichtung nach Anspruch 7, wobei das Geflecht sich in Nachbarschaft zu dem distalen Bereich befindet.

9. Vorrichtung nach Anspruch 7, wobei das Geflecht sich in Nachbarschaft zu dem wulstförmigen Teil befindet.

10. Vorrichtung nach Anspruch 7, wobei das Geflecht mehrere Fasern aufweist, die miteinander verflochtenen sind, und wobei mindestens eine der Fasern ein strahlenundurchlässiges Material aufweist.

11. Vorrichtung nach Anspruch 1, wobei das wulstförmige Teil mit dem distalen Bereich einstückig ist.

12. Vorrichtung nach Anspruch 1, wobei das wulstförmige Teil mehrere Untereinheiten aufweist.

13. Vorrichtung nach Anspruch 1 oder 5, wobei die Hülle eine oder mehrere Längsnuten aufweist.

14. Vorrichtung nach Anspruch 13, wobei die eine oder die mehreren Nuten sich in Nachbarschaft zu dem wulstförmigen Teil befinden.

15. Vorrichtung nach Anspruch 13, wobei die eine oder die mehreren Nuten sich proximal bezüglich des wulstförmigen Teils erstrecken.

16. Vorrichtung nach Anspruch 1, wobei der distale Bereich eine Stützspirale aufweist.

## Revendications

1. Dispositif destiné à être utilisé avec un filtre de protection embolique (12), comprenant :
une gaine oblongue (10) avec une zone proximale (22), une zone distale (24), une lumière s'étendant au moins en partie au travers de celle-ci, et une embouchure distale (31) adjacente à la zone distale ;
l'embouchure distale étant expansible et prévue pour passer d'une configuration initiale à une configuration élargie ;
**caractérisé en ce que** la zone distale comprend un élément renflé (26) dans la configuration initiale.

2. Dispositif selon la revendication 1, où l'élément renflé présente un bord proximal chanfreiné et un bord distal chanfreiné.

3. Dispositif selon la revendication 1, où au moins une partie de la zone distale a une épaisseur accrue.

4. Dispositif selon la revendication 3, où la partie de la zone distale avec une épaisseur accrue est adjacente à l'élément renflé.

5. Dispositif selon la revendication 1, où la lumière adjacente à la zone distale est évasée, de telle manière que la gaine présente un premier diamètre intérieur adjacent à la zone proximale et un deuxième diamètre intérieur adjacent à la zone distale, le deuxième diamètre intérieur étant supérieur au premier diamètre intérieur.

6. Dispositif selon la revendication 1 ou la revendication 5, où la zone proximale du tube est définie par une première tige tubulaire et la zone distale du tube par une deuxième tige tubulaire fixée à la première tige.

7. Dispositif selon la revendication 1 ou la revendication 5, où la gaine comporte une tresse.

8. Dispositif selon la revendication 7, où la tresse est adjacente à la zone distale.

9. Dispositif selon la revendication 7, où la tresse est adjacente à l'élément renflé.

10. Dispositif selon la revendication 7, où la tresse comprend une pluralité de fibres tressées entre elles, et où au moins une des fibres contient un matériau radio-opaque.

11. Dispositif selon la revendication 1, où l'élément renflé est incorporé à la zone distale.

12. Dispositif selon la revendication 1, où l'élément renflé comprend une pluralité de sous-unités.

13. Dispositif selon la revendication 1 ou la revendication 5, où la gaine présente une ou plusieurs rainures longitudinales.

14. Dispositif selon la revendication 13, où la ou les rainures sont adjacentes à l'élément renflé.

15. Dispositif selon la revendication 13, où la ou les rainures s'étendent proximalement à l'élément renflé.

16. Dispositif selon la revendication 1, où la région distale comporte une bobine de support.
